(19) Europäisches Patentamt
European Patent Office
Office européen des brevets

(11) EP 4 193 995 A1

(12) **EUROPEAN PATENT APPLICATION**
published in accordance with Art. 153(4) EPC

(43) Date of publication:
**14.06.2023 Bulletin 2023/24**

(21) Application number: **21855512.6**

(22) Date of filing: **10.08.2021**

(51) International Patent Classification (IPC):
***A61K 31/5025*** *(2006.01)* ***A61P 25/00*** *(2006.01)*
***A61P 29/00*** *(2006.01)*

(52) Cooperative Patent Classification (CPC):
**A61K 31/5025; A61P 25/00; A61P 29/00**

(86) International application number:
**PCT/CN2021/111726**

(87) International publication number:
**WO 2022/033460 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.08.2020 CN 202010796482**
**19.03.2021 CN 202110295378**

(71) Applicants:
- **Jiangsu Hengrui Pharmaceuticals Co., Ltd.**
  **Lianyungang, Jiangsu 222047 (CN)**
- **Reistone Biopharma Company Limited**
  **Shanghai 201210 (CN)**

(72) Inventors:
- **WANG, Min**
  **Shanghai 201210 (CN)**
- **WU, Yihan**
  **Shanghai 201210 (CN)**
- **ZHOU, Ling**
  **Shanghai 201210 (CN)**
- **LU, Zailian**
  **Shanghai 201210 (CN)**
- **LI, Baoping**
  **Shanghai 201210 (CN)**

(74) Representative: **Regimbeau**
**20, rue de Chazelles**
**75847 Paris Cedex 17 (FR)**

# (54) USE OF BTK INHIBITORS IN THE TREATMENT OF DISEASES

(57) The present disclosure relates to the use of BTK inhibitors in the treatment of diseases. Specifically, the present disclosure relates to the use of BTK inhibitors in the preparation of drugs for the prevention or treatment of neuromyelitis optica spectrum disorders.

EP 4 193 995 A1

## Description

### FIELD OF THE INVENTION

The present disclosure belongs to the field of medicine, and relates to a use of a BTK inhibitor for treating diseases.

### BACKGROUND OF THE INVENTION

Neuromyelitis optica (NMO, also known as Devic syndrome) is a rare, chronic, autoimmune, inflammatory central nervous system (CNS) demyelinating disease that affects the optic nerve and spinal cord. It is characterized by recurrent episodes of optic neuritis and longitudinally extensive transverse myelitis (LETM), which may occur simultaneously or independently.

Clinical features of NMO include optic neuritis with vision loss leading to blindness, as well as eye pain, optic atrophy, and central scotoma. Spinal cord involvement includes paraparesis or quadriparesis, radicular pain, paroxysmal tonic spasms, nausea, intractable hiccups, vomiting, vertigo, bladder dysfunction, and Lhermitte's phenomenon. Clinical features associated with lesions other than the optic nerve and spinal cord lesions may include excessive sleepiness associated with hypothalamic-pituitary axis insufficiency, hyponatremia and hyperprolactinemia, and reversible posterior encephalopathy syndrome. Up to 90% of NMO patients have recurrent optic neuritis (ON) and myelitis rather than a monophasic course. 60% of patients relapse within 1 year after onset, and 90% of patients relapse within 3 years after onset.

Previously, NMO was considered a subtype of multiple sclerosis (MS), which has symptoms overlapping with NMO. However, NMO has now been proven to be a unique demyelinating disease different from MS. Unlike MS, recovery between episodes of NMO is incomplete, with each succeeding episode leading to increased disability. The antigenic target, aquaporin 4 (AQP4), was not identified until 2004. The two diseases could be reliably distinguished by detection of antibodies to AQP4. Later, an update of the diagnostic guidelines classified antibody-negative and antibody-positive NMOs into neuromyelitis optica spectrum disorders (NMOSD). Although case series studies and observational studies have shown that patients benefit from immunotherapy, only 2 therapies (Eculizumab and Satralizumab) have been approved to treat NMOSD to date.

AQP4 is the most widely expressed aquaporin in the brain, spinal cord and optic nerve. Experimental data showed that antibodies to AQP4 induced the production of interleukin 6 (IL-6) in AQP4-expressing astrocytes, and IL-6 signaling to endothelial cells reduced blood-brain barrier function. Once the antibody to AQP4 binds to the ectodomain of the AQP4 receptor, it not only causes the internalization of the glutamate transporter EAAT-2, but also causes complement and cell-mediated astrocyte damage. Astrocytes eventually no longer support surrounding cells such as oligodendrocytes and neurons. Then granulocytic infiltration occurs, with oligodendrocyte injury and demyelination.

Immune cells can usually be divided into T cells and B cells. The main function of B cells is to secrete various antibodies to help the body resist various foreign invasions. Bruton's tyrosine kinase (BTK) is one of the members of the tyrosine kinase subfamily, and belongs to the Tec family of kinases. It is mainly expressed in B cells, and distributed in the lymphatic system, hematopoietic and blood systems. B cell receptor (BCR) plays a crucial role in regulating the proliferation and survival of various lymphomas including chronic lymphocytic leukemia (CLL) and subtypes of non-Hodgkin's lymphoma (NHL), mantle cell lymphoma (MCL), and diffuse large B-cell lymphoma (DLBCL). In addition, the role of B cells in the pathogenesis of rheumatoid arthritis, systemic lupus erythematosus, multiple sclerosis, and other immune diseases has been clinically confirmed. Bruton's tyrosine kinase (BTK) is a key protein kinase in the BCR signaling pathway. It can regulate the maturation and differentiation of normal B cells, and is also closely related to a variety of B cell lymphoid tissue disorders. Therefore, small-molecule inhibitors targeting BTK may provide benefits in the treatment of B-cell malignancy and autoimmune disease.

WO2016007185 relates to a compound of formula (I), namely (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-*d*]pyridazin-7-one. The compound is a novel BTK kinase inhibitor, which has improved kinase selectivity, clinical efficacy or indications, and safety. Its structure is as follows:

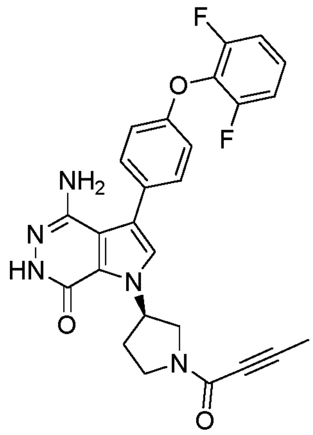

I

The present disclosure provides a use of a BTK inhibitor for treating or preventing diseases, and shows good therapeutic effect.

## SUMMARY OF THE INVENTION

In an aspect, the present disclosure provides a use of a BTK inhibitor in the preparation of a medicament for preventing or treating neuromyelitis optica spectrum disorder.

In another aspect, the present disclosure provides a use of a BTK inhibitor in the preparation of a medicament for preventing relapse of neuromyelitis optica spectrum disorder.

In another aspect, the present disclosure provides a use of a BTK inhibitor in the preparation of a medicament for treating stable phase of neuromyelitis optica spectrum disorder.

The stable phase of the neuromyelitis optica spectrum disorder refers to the phase when the condition of the neuromyelitis optica spectrum disorder is alleviated after the rescue therapy is carried out in the acute attack phase. For example, when treating with the BTK inhibitor, the dose of the maintenance drug such as immunosuppressant (such as tacrolimus, azathioprine, mycophenolate) and/or oral corticosteroid must be stable or in the process of reduction, and the rescue therapy (such as IV glucocorticoid, plasma exchange, and/or intravenous immunoglobulin) must be completed for more than 1 month.

In some embodiments, the BTK inhibitor is selected from the group consisiting of ibrutinib, acalabrutinib, GS-4059, spebrutinib, BGB-3111, FIM71224, zanubrutinib, ARQ531, BI-BTK1, vecabrutinib and a compound of formula (I) or a pharmaceutically acceptable salt thereof,

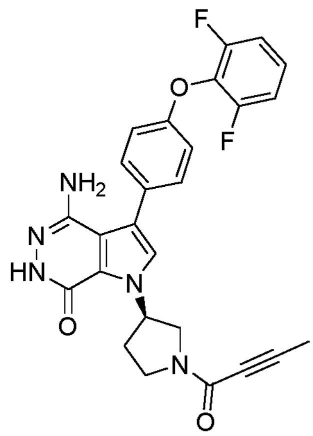

I .

In some embodiments, the BTK inhibitor is the compound of formula (I) or a pharmaceutically acceptable salt thereof,

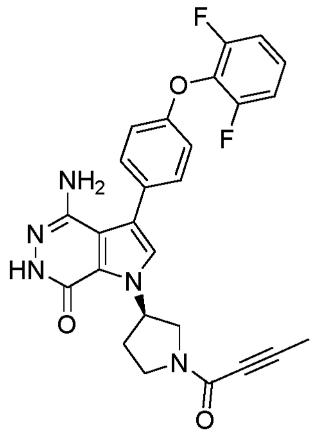

I

The compound of the present disclosure can also exists in different tautomeric forms, all tautomeric forms are within the scope of the present disclosure. The term "tautomer" or "tautomeric form" refers to structural isomers of different energies that can interconvert via a low energy barrier. For example, proton tautomer (also known as prototropic tautomer) includes interconversion via migration of a proton, such as keto-enol and imine-enamine isomerizations. An example of a lactam-lactim equilibrium is an equilibrium between A and B as shown below.

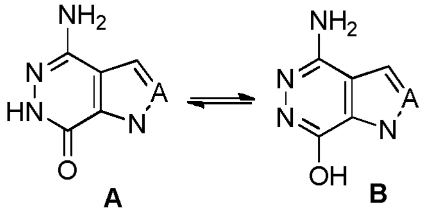

A B

In some embodiments, the compound of formula (I) of the present disclosure can be drawn as type A or type B. The naming of compounds does not exclude any tautomers. The clinical manifestations of the neuromyelitis optica spectrum disorder described in the present disclosure include optic neuritis, myelitis, area postrema syndrome, brainstem syndrome, diencephalic syndrome, cerebral syndrome, and the like.

In some embodiments, the neuromyelitis optica spectrum disorder is an AQP4-IgG positive neuromyelitis optica spectrum disorder.

In some embodiments, the dose of the BTK inhibitor is 1 to 1000 mg, for example, can be 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg. The administration frequency can be once a day, twice a day, three times a day, once every two days, once every three days, once a week, and the like.

In some embodiments, the administration frequency of the BTK inhibitor is once a day, and the dose is 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg each time.

In some embodiments, the administration frequency of the BTK inhibitor is twice a day, and the dose is 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg each time.

The pharmaceutically acceptable salt of the drug of the present disclosure can be hydrochloride, phosphate,

hydrophosphate, sulfate, bisulfate, sulfite, acetate, oxalate, malonate, valerate, glutamate, oleate, palmitate, stearate, laurate, borate, *p*-toluenesulfonate, mesylate, isethionate, maleate, malate, tartrate, benzoate, pamoate, salicylate, vanillate, mandelate, succinate, gluconate, lactobionate, lauryl sulfonate, and the like.

The BTK inhibitor of the present disclosure can also be administered in combination with other drugs. The combined administration mode is selected from the group consisiting of simultaneous administration, co-administration after separate formulation, and sequential administration after separate formulation.

The administration route of the drug of the present disclosure is selected from the group consisiting of oral administration, parenteral administration and transdermal administration. The parenteral administration includes, but is not limited to, intravenous injection, subcutaneous injection, and intramuscular injection.

The disclosure further relates to a method for treating neuromyelitis optica spectrum disorder, comprising administering a BTK inhibitor to a patient.

In some embodiments, the present disclosure relates to a method for treating neuromyelitis optica spectrum disorder, comprising administering the compound of formula (I) or a pharmaceutically acceptable salt thereof to a patient.

The present disclosure further relates to the compound of formula (I) or a pharmaceutically acceptable salt thereof for use in treating neuromyelitis optica spectrum disorder.

The present disclosure further relates to a method for preventing relapse of neuromyelitis optica spectrum disorder, comprising administering a BTK inhibitor to a patient.

In some embodiments, the present disclosure relates to a method for preventing relapse of neuromyelitis optica spectrum disorder, comprising administering the compound of formula (I) or a pharmaceutically acceptable salt thereof to a patient.

The present disclosure further relates to a BTK inhibitor for use in preventing relapse of neuromyelitis optica spectrum disorder.

In some embodiments, the present disclosure relates to the compound of formula (I) or a pharmaceutically acceptable salt thereof for use in preventing relapse of neuromyelitis optica spectrum disorder.

The present disclosure further relates to a method for treating stable phase of neuromyelitis optica spectrum disorder, comprising administering a BTK inhibitor to a patient.

In some embodiments, the present disclosure relates to a method for treating stable phase of neuromyelitis optica spectrum disorder, comprising administering the compound of formula (I) or a pharmaceutically acceptable salt thereof to a patient.

The present disclosure further relates to a BTK inhibitor for use in treating stable phase of neuromyelitis optica spectrum disorder.

In some embodiments, the present disclosure relates to the compound of formula (I) or a pharmaceutically acceptable salt thereof for use in treating stable phase of neuromyelitis optica spectrum disorder.

The present disclosure also relates to a pharmaceutical composition comprising the compound of formula (I) or a pharmaceutically acceptable salt thereof, and one or more pharmaceutically acceptable carriers, excipients and diluents. The pharmaceutical composition can be formulated into any pharmaceutically acceptable dosage form. For example, the pharmaceutical composition can be formulated into a tablet, capsule, pill, granule, solution, suspension, syrup, injection (including injection solution, sterile powder for injection and concentrated solution for injection), suppository, inhalant or spray.

The present disclosure also provides a pharmaceutical packaging box, in which the pharmaceutical composition of the compound of formula (I) or a pharmaceutically acceptable salt thereof of the present disclosure is packaged.

A "therapeutically effective amount" refers to the amount of a therapeutic agent that produces the desired effect for which it is administered. In some embodiments, the term refers to an amount sufficient to treat a disease, disorder and/or condition when administered according to a regimen to a population suffering from or susceptible to the disease, disorder and/or condition. In some embodiments, a therapeutically effective amount is an amount that reduces the incidence and/or severity of, and/or delays the onset of, one or more symptoms of a disease, disorder and/or condition. Those of ordinary skill in the art will appreciate that the term "therapeutically effective amount" actually does not need to achieve successful treatment in a particular individual. Conversely, a therapeutically effective amount can be an amount that, when administered to a patient in need of such treatment, provides a specific desired pharmacological response in a substantial number of subjects. In some embodiments, a reference to a therapeutically effective amount can refer to an amount as measured in one or more particular tissues (such as tissues affected by a disease, disorder or condition) or fluid (such as blood, saliva, serum, sweat, tears, urine, and the like). Those of ordinary skill in the art will appreciate that, in some embodiments, a therapeutically effective amount of a particular agent or therapy can be formulated and/or administered in a single dose. In some embodiments, a therapeutically effective agent can be formulated and/or administered in multiple doses, for example, as part of a regimen.

Average annualized relapse rate (ARR) = total number of episodes of subjects / (number of subjects * observation time).

The achievable effect of the compound of formula (I) or a pharmaceutically acceptable salt of the present disclosure in treating neuromyelitis optica spectrum disorder is that the subject's ARR<2.1 after baseline medication.

The achievable effect of the compound of formula (I) or a pharmaceutically acceptable salt of the present disclosure in treating neuromyelitis optica spectrum disorder is that the subject's ARR after baseline medication can be selected from the group consisiting of 0, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 and 2.0.

## DETAILED DESCRIPTION OF THE INVENTION

Example 1: Clinical research of the compound of formula (I) in treating neuromyelitis optica spectrum disorder (NMOSD)

1. Experimental drug

Tablets of the compound of formula (I), specification: 100 mg/tablet.

2. Enrollment criteria

1) Male or female from 18 to 75 years old (including 18 and 75 years old);
2) Patients diagnosed with AQP4-IgG positive NMOSD according to the 2015 IPND diagnostic criteria;
3) Requirements for history of relapse: 2 or more relapses of NMOSD requiring rescue therapy within 1 year before baseline, the rescue therapy can include IV glucocorticoid, plasma exchange, and/or intravenous immune globulin (IVIG);
4) Subjects must have been on stable treatment (if any) for more than 1 month before starting test drug treatment, defined as follows:
the amount of immunosuppressant (such as tacrolimus, azathioprine, mycophenolate) and/or oral corticosteroid must be stable or in the process of reduction, and the acute phase rescue therapy (such as IV glucocorticoid, plasma exchange, and/or intravenous immunoglobulin) must be completed for more than 1 month.

2. Administration regimen

The test drug was administered to the qualified subjects in the morning on an empty stomach, once a day, one tablet at a time, and 100 mg per tablet.

3. Experimental results

At present, the study has completed the enrollment of all 10 subjects, and the average annualized relapse rate (ARR = the total number of episodes of subjects / (number of subjects*observation time)) of all subjects before enrollment was 2.1. By now, an annualized relapse rate of 0.7 for existing subjects after baseline medication has been observed. A 42-year-old female patient with a 7-year history of NMOSD had severe symptoms (unable to stand) before participating in the trial. After administering the compound of formula (I) for 4 weeks, the patient obtained significantly improved mobility, and could stand up and walk several steps with assistance. At the 7$^{th}$ week of medication, the patient could walk more than 20 meters with assistance, and could substantially take care of herself.

## Claims

1. Use of a BTK inhibitor in the preparation of a medicament for preventing or treating neuromyelitis optica spectrum disorder.

2. Use of a BTK inhibitor in the preparation of a medicament for preventing relapse of neuromyelitis optica spectrum disorder.

3. Use of a BTK inhibitor in the preparation of a medicament for treating stable phase of neuromyelitis optica spectrum disorder.

4. The use according to any one of claims 1 to 3, wherein the neuromyelitis optica spectrum disorder is an AQP4-IgG positive neuromyelitis optica spectrum disorder.

5. The use according to any one of claims 1 to 4, wherein the dose of the BTK inhibitor is 1 to 1000 mg.

6. The use according to any one of claims 1 to 4, wherein the administration frequency of the BTK inhibitor is selected from the group consisiting of once a day, twice a day, three times a day, once every two days, once every three days, and once a week.

7. The use according to any one of claims 1 to 4, wherein the administration frequency of the BTK inhibitor is once a day, and the dose is 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg each time.

8. The use according to any one of claims 1 to 4, wherein the administration frequency of the BTK inhibitor is twice a day, and the dose is 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg each time.

9. The use according to any one of claims 1 to 8, wherein the BTK inhibitor is selected from the group consisiting of ibrutinib, acalabrutinib, GS-4059, spebrutinib, BGB-3111, FIM71224, zanubrutinib, ARQ531, BI-BTK1, vecabrutinib and a compound of formula (I) or a pharmaceutically acceptable salt thereof,

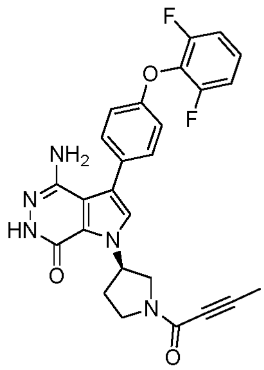

I .

10. The use according to any one of claims 1 to 8, wherein the BTK inhibitor is a compound of formula (I) or a pharmaceutically acceptable salt thereof,

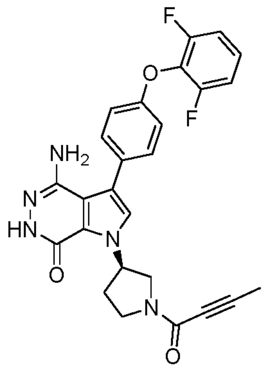

I .

**INTERNATIONAL SEARCH REPORT**

International application No.
**PCT/CN2021/111726**

**A. CLASSIFICATION OF SUBJECT MATTER**

A61K 31/5025(2006.01)i; A61P 25/00(2006.01)i; A61P 29/00(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNABS, CNKI, 万方, DWPI, VEN, STNext, WEB OF SCIENCE, 读秀学术搜索, 百度学术搜索: 江苏恒瑞医药, 瑞石生物医药, BTK抑制剂, 布鲁顿酪氨酸蛋白激酶抑制剂, Bruton酪氨酸蛋白激酶抑制剂, 视神经脊髓炎, 视神经脊髓炎谱系疾病, Bruton's tyrosine kinase (BTK) inhibitor, ibrutinib, acalabrutinib, GS-4059, spebrutinib, BGB-3111, FIM71224, zanubrutinib, ARQ531, BI-BTK1, vecabrutinib, 1858206-58-2, STN structural formula search, neuromyelitis optica, NMO, neuromyelitis optica spectrum disorders, NMOSD

**C. DOCUMENTS CONSIDERED TO BE RELEVANT**

| Category* | Citation of document, with indication, where appropriate, of the relevant passages | Relevant to claim No. |
|---|---|---|
| X | 王炜 (WANG, Wei). "Btk/NF-κB 信号通路及相关细胞因子在视神经脊髓炎谱系疾病中作用机制探讨 (The Role of Btk/NF-κB Signaling Pathway and Related Cytokines in the Pathogenesis of Optic Neuromyelitis Spectrum Disease)" *《中国优秀博硕士学位论文全文数据库(硕士)医药卫生科技辑》 (Chinese Master's Theses Full-text Database, Medicine & Public Health),* No. 01, 15 January 2020 (2020-01-15), abstract, and pp. 43-48 | 1-9 |
| Y | 王炜 (WANG, Wei). "Btk/NF-κB 信号通路及相关细胞因子在视神经脊髓炎谱系疾病中作用机制探讨 (The Role of Btk/NF-κB Signaling Pathway and Related Cytokines in the Pathogenesis of Optic Neuromyelitis Spectrum Disease)" *《中国优秀博硕士学位论文全文数据库(硕士)医药卫生科技辑》 (Chinese Master's Theses Full-text Database, Medicine & Public Health),* No. 01, 15 January 2020 (2020-01-15), abstract, and pp. 43-48 | 9-10 |
| Y | CN 106573001 A (ETERNITY BIOSCIENCE, INC.) 19 April 2017 (2017-04-19) description page 1 paragraph 0001, page 56 embodiment 117 | 9-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 October 2021** | **10 November 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
**PCT/CN2021/111726**

**C. DOCUMENTS CONSIDERED TO BE RELEVANT**

| Category* | Citation of document, with indication, where appropriate, of the relevant passages | Relevant to claim No. |
|---|---|---|
| Y | WO 2017118277 A1 (JIANGSU HENGRUI MEDICINE CO., LTD.) 13 July 2017 (2017-07-13) claims 1, 7-9 | 9-10 |
| Y | CN 111499642 A (JIANGSU HENGRUI MEDICINE CO., LTD.) 07 August 2020 (2020-08-07) claim 1, description paragraphs 0002-0004 | 9-10 |
| A | CN 110772521 A (SUZHOU ASCENTAGE PHARMA CO., LTD.) 11 February 2020 (2020-02-11) description, paragraph 0070 | 1-10 |
| A | WO 2019126759 A1 (CONVELO THERAPEUTICS, INC.) 27 June 2019 (2019-06-27) claims 16-26 | 1-10 |

Form PCT/ISA/210 (second sheet) (January 2015)

**INTERNATIONAL SEARCH REPORT**
**Information on patent family members**

International application No.
**PCT/CN2021/111726**

| Patent document cited in search report | Publication date (day/month/year) | Patent family member(s) | Publication date (day/month/year) |
|---|---|---|---|
| CN 106573001 A | 19 April 2017 | TW 201625626 A | 16 July 2016 |
| | | US 2017152264 A1 | 01 June 2017 |
| | | US 2018251465 A1 | 06 September 2018 |
| | | ZA 201700115 B | 27 May 2020 |
| | | KR 102073797 B1 | 05 February 2020 |
| | | AU 2014400628 B2 | 02 May 2019 |
| | | JP 6409116 B2 | 17 October 2018 |
| | | EP 3166608 A1 | 17 May 2017 |
| | | RU 2017103757 A3 | 07 August 2018 |
| | | ES 2706745 T3 | 01 April 2019 |
| | | RU 2674701 C2 | 12 December 2018 |
| | | KR 20170023156 A | 02 March 2017 |
| | | US 10323037 B2 | 18 June 2019 |
| | | CA 2953798 A1 | 14 January 2016 |
| | | EP 3166608 A4 | 22 November 2017 |
| | | CN 109970741 A | 05 July 2019 |
| | | CA 2953798 C | 11 June 2019 |
| | | AU 2014400628 A1 | 19 January 2017 |
| | | MX 2017000331 A | 25 August 2017 |
| | | CN 109970741 B | 28 July 2020 |
| | | EP 3166608 B1 | 12 December 2018 |
| | | US 9951077 B2 | 24 April 2018 |
| | | BR 112017000470 A2 | 07 November 2017 |
| | | CN 106573001 B | 29 January 2019 |
| | | WO 2016007185 A1 | 14 January 2016 |
| | | TW I711619 B | 01 December 2020 |
| | | PL 3166608 T3 | 31 July 2019 |
| | | JP 2017522302 A | 10 August 2017 |
| | | RU 2017103757 A | 07 August 2018 |
| WO 2017118277 A1 | 13 July 2017 | KR 20180099787 A | 05 September 2018 |
| | | US 2019010161 A1 | 10 January 2019 |
| | | AU 2016384921 C1 | 20 May 2021 |
| | | TW 201725207 A | 16 July 2017 |
| | | EP 3372607 B1 | 28 April 2021 |
| | | RU 2018125277 A | 07 February 2020 |
| | | AU 2016384921 A1 | 26 July 2018 |
| | | CN 107406453 A | 28 November 2017 |
| | | RU 2018125277 A3 | 28 February 2020 |
| | | CN 107406453 B | 28 December 2018 |
| | | RU 2728827 C2 | 31 July 2020 |
| | | CA 3009256 A1 | 13 July 2017 |
| | | EP 3372607 A1 | 12 September 2018 |
| | | AU 2016384921 B2 | 15 October 2020 |
| | | BR 112018012106 A2 | 04 December 2018 |
| | | US 10626116 B2 | 21 April 2020 |
| | | JP 2019500357 A | 10 January 2019 |
| | | EP 3372607 A4 | 03 October 2018 |
| | | MX 2018008131 A | 03 September 2018 |
| | | HK 1243419 B | 22 November 2019 |
| CN 111499642 A | 07 August 2020 | None | |

Form PCT/ISA/210 (patent family annex) (January 2015)

**INTERNATIONAL SEARCH REPORT**
**Information on patent family members**

International application No.
**PCT/CN2021/111726**

| Patent document cited in search report | | | Publication date (day/month/year) | Patent family member(s) | | | Publication date (day/month/year) |
|---|---|---|---|---|---|---|---|
| CN | 110772521 | A | 11 February 2020 | BR | 112020019189 | A2 | 09 February 2021 |
| | | | | EP | 3672639 | A4 | 30 September 2020 |
| | | | | TW | 202007396 | A | 16 February 2020 |
| | | | | EP | 3672639 | A1 | 01 July 2020 |
| | | | | AU | 2019315487 | A1 | 01 October 2020 |
| | | | | CA | 3094452 | A1 | 06 February 2020 |
| | | | | EA | 202091965 | A1 | 14 May 2021 |
| | | | | WO | 2020024916 | A1 | 06 February 2020 |
| | | | | KR | 20200138724 | A | 10 December 2020 |
| | | | | US | 2020222393 | A1 | 16 July 2020 |
| WO | 2019126759 | A1 | 27 June 2019 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016007185 A